# EUROPEAN PATENT APPLICATION

(11) **EP 1 160 248 A1**
(43) Date of publication of application: **05.12.2001**
(21) Application number: 01304626.3
(22) Date of filing: 25.05.2001
(51) Int. Cl.: C07D 413/14, A61K 31/4155, A61K 31/506

(54) **N-(Isoxazol-5-yl)-sulfonamide derivatives and their use as endothelin antagonists**

(30) Priority: 31.05.2000 GB 0013368; 26.07.2000 GB 0018356
(71) Applicant: PFIZER INC., New York, N.Y. 10017 (US); Pfizer Limited, Sandwich, Kent CT13 9NJ (GB)
(72) Inventor: Banks, Bernard Joseph, Sandwich, Kent CT13 9NJ (GB); Chubb, Nathan Anthony Logan, Sandwich, Kent CT13 9NJ (GB); Eshelby, James John, Sandwich, Kent CT13 9NJ (GB); Pacey, Michael Stephen, Sandwich, Kent CT13 9NJ (GB); Schulz, Darren John, Sandwich, Kent CT13 9NJ (GB)
(74) Representative: Simpson, Alison Elizabeth Fraser

(57) **Abstract**

Isoxazoles of formula (I) below have good affinity for endothelin receptors, and are selective for ET_{A} over ET_{B}. 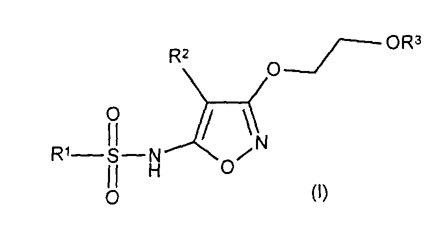 wherein
R¹ is either
   a)a phenyl group; or
   b)an optionally benzo-fused 5- or 6-membered heterocyclic group with one to three heteroatoms in the heterocyclic ring, which heteroatoms are independently selected from N, O and S; or
   CHR⁶CHR⁷Ph, CR⁶:CR⁷Ph;
   each of which groups (a) (b) and (c) is optionally substituted by up to three substituents each independently selected from:
   i)halo;
   ii)C₁₋₆ alkyl optionally substituted by OH, halogen, NR⁴R⁵ OCOR⁴ or CO₂R⁴;
   iii)CN;
   iv)O(C₁₋₈ alkyl optionally substituted by one or more halogen); and
   v)CO₂R⁴;
R² is aryl¹ or het¹,
R³ is H, C₁₋₆ alkyl, C(O)R⁴, CONHaryl¹, CONHhet¹, aryl¹ and het¹
Such compounds are useful in the treatment of conditions mediated by endothelin.

## Description

This invention relates to isoxazole derivatives useful in the treatment of a variety of conditions mediated by endothelin and to pharmaceutical formulations containing such compounds useful for the treatment of humans and non-human mammals.

Endothelin (ET) is a potent vasoconstrictor synthesised and released by endothelial cells. There are three distinct isoforms of ET: ET-1, ET-2 and ET-3, all being 21-amino acid peptides and herein the term 'endothelin' refers to any or all of the isoforms. Two receptor subtypes, ET_{A} and ET_{B} have been pharmacologically defined (see for example H. Arai et al., *Nature,* **348,** 730 , 1990) and further subtypes have recently been reported. Stimulation of ET_{A} promotes vasoconstriction and stimulation of ET_{B} receptors causes either vasodilation or vasoconstriction. The main effects of ET are observed in the cardiovascular system, particularly in the coronary, renal, cerebral and mesenteric circulation, and the effects of endothelin are often long-lasting. Stimulation of ET receptors also mediate further biological responses in cardiovascular and non-cardiovascular tissues such as cell proliferation and matrix formation.

Increased circulating levels of endothelin have been observed in patients who have undergone percutaneous transluminal coronary angioplasty (PTCA) (A. Tahara et al., *Metab*. *Clin. Exp.* **40**, 1235, 1991) and ET-1 has been found to induce neointimal formation in rats after balloon angioplasty (S.Douglas et al., *J. Cardiovasc. Pharm.,* **22** (Suppl 8), 371, 1993). The same workers have found that an endothelin antagonist, SB-209670, causes a 50% reduction in neointimal formation relative to control animals (S. Douglas et al., *Circ. Res,* 75, 1994). Antagonists of the endothelin receptor may thus be useful in preventing restenosis post PTCA. The ET_{A/B} receptor antagonist Bosentan reportedly decreased blood pressure in hypertensive patients (H. Krum et al., New Eng. J. Med. (1998) **338,** 784-790). Antagonists of ET_{B} receptors such as BQ-788 have been demonstrated to increase peripheral resistance in man (Hypertension (1999) 33, 581-585). Thus ET_{A}-selective receptor antagonists are of benefit in hypertension.

Endothelin-1 is produced in the human prostate gland and endothelin receptors have been identified in this tissue (Y. Saita et al., Eur. J. Pharmacol. (1988) **349,** 123-128). Since endothelin is a contractile and proliferative agent, endothelin antagonists are useful in the treatment of benign prostate hypertrophy.

There is widespread localisation of endothelin and its receptors in the central nervous system and cerebrovascular system (R. K. Nikolov et al., *Drugs of Today,* **28**(5), 303, 1992) with ET being implicated in cerebral vasospasm, cerebral infarcts, septic shock, myocardial infarction and neuronal death.

Elevated levels of endothelin have also been observed in patients with:
- recurrent airway obstruction (*Pulm. Pharm. Ther.,* (1998) **11**: 231-235);
- asthma (*Am*. *J. Resp. Crit. Care Med.,* (1995) **151**:1034-1039);
- acute renal failure (K. Tomita, et al, *Med. Philos.* (1994) **13(1),** 64-66);
- chronic renal failure (F. Stockenhuber et al., *Clin. Sci.* (Lond.), **82,** 255, 1992);
- ischaemic Heart Disease (M. Yasuda, *Am. Heart J.,* **119,** 801, 1990);
- stable or unstable angina (J. T. Stewart, *Br. Heart J.,* **66**, *7* 1991);
- pulmonary hypertension (D. J. Stewart et al., *Ann. Internal Medicine,* **114,** 464, 1991);
- congestive heart failure (R. J. Rodeheffer et al., *Am. J. Hypertension,* **4,** 9A, 1991);
- preeclampsia (B. A. Clark et al., *Am. J. Obstet. Gynecol*., **166**, 962, 1992);
- diabetes (A. Collier et al., *Diabetes Care,* **15** (8), 1038, 1992);
- Crohn's disease (S. H. Murch et al., *Lancet,* **339,** 381, 1992); and
- atherosclerosis (A. Lerman et al., *New Eng. J. Med*., **325,** 997, 1991).

In every case the disease state associated with the physiologically elevated levels of endothelin is potentially treatable with a substance which decreases the effect of endothelin, such as an endothelin receptor antagonist, or a compound which binds endothelin such that it reduces the effective concentration thereof at the endothelin receptors.

Compounds that antagonise the ET_{A} receptor to a greater extent than the ET_{B} receptor are preferred as ET_{A} receptors are predominantly present in vascular smooth muscles. Blockade of ET_{B} receptor activation may reverse endothelial dependent vasodilation which is beneficial in hypertension. ET may also mediate regeneration of damaged tissue via the ET_{B} receptor, such as proximal tubule cells in the kidney. Thus blockade of ET_{B} receptors, e.g. with a non-selective ET antagonist could inhibit tissue repair. ET_{B} receptors are also involved in the clearance of ET from the systemic circulation. Increased levels of ET are generally considered detrimental. Rises in circulating levels have been observed with non-selective ET antagonists. Treatment with selective ET_{A} receptor antagonists are not likely to induce such rises in circulating levels.

There are a number of publications relating to N-(pyrimidin-4-yl)sulphonamide derivatives having endothelin binding / antagonist activity, for example EP-A-0743307, EP-A-0658548, EP-A-0633259, EP-A-0882719, WO-A-96/20177, EP-A-0801062, WO-A-97/09318, EP-A-0852226, EP-A-0768304, WO-A-96/19459, WO-A-98/03488, WO-A-98/57938, WO-A-99/36408, WO-A-01/17976 and EP-A-0713875.

Various *N*-4-pyrimidinyl sulphonamide derivatives possessing endothelin antagonist activity are described in EP-A-0882719, JP-A-09059160, JP-A-10194972 and JP-A-10226649.

International Patent Application publication number WO-A-96/19455 discloses phenyl and pyridin-4-yl sulphonamides as endothelin antagonists.

International Patent Application publication number WO-A-97/11942 discloses various (4-arylthioisoxazol-3-yl)sulphonamides, with an aldehyde moiety linked to the 5-position of the isoxazole ring, as selective ET_{B} receptor selective antagonists.

We have unexpectedly found that isoxazoles of formula (I) below have good affinity for endothelin receptors, and are selective for ET_{A} over ET_{B}. 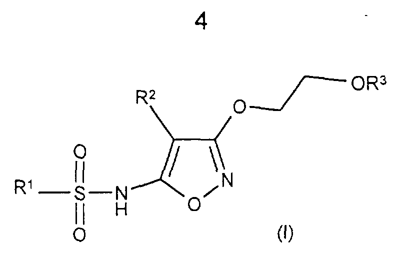 wherein
R¹ is either
   a) a phenyl group; or
   b) an optionally benzo-fused 5- or 6-membered heterocyclic group with one to three heteroatoms in the heterocyclic ring, which heteroatoms are independently selected from N, O and S; or
   c) CHR⁶CHR⁷Ph, CR⁶:CR⁷Ph;
   each of which groups (a) (b) and (c) is optionally substituted by up to three substituents each independently selected from:
   i) halo;
   ii) C₁₋₆ alkyl optionally substituted by OH, halogen, NR⁴R⁵ OCOR⁴ or CO₂R⁴;
   iii) CN;
   iv) O(C₁₋₆ alkyl optionally substituted by one or more halogen); and
   v) CO₂R⁴;
R⁴ and R⁵ are each independently H or C₁₋₆ alkyl optionally substituted by one or more halo,
R⁶ and R⁷ are each independently H or C₁₋₃ alkyl,
R² is aryl¹ or het¹,
R³ is H, C₁₋₆ alkyl, C(O)R⁴, CONHaryl¹, CONHhet¹, aryl¹ and het¹,
aryl¹ is a phenyl or naphthyl group optionally substituted by up to three substituents each independently selected from C₁₋₃ alkyl, CF₃, halo, C₁₋₃ alkoxy, OCF₃, OH, NO₂, CN, NR⁴R⁵, COR⁴, CO₂R⁴, CONR⁴R⁵, S(O)ₚ(C₁₋₃ alkyl), CH₂NR⁴R⁵, NR⁴COR⁵, COCF₃, CH₂OH, S(O)ₚCF₃, C(=NH)NH₂, C₂₋₃ alkynyl, C₂₋₃ alkenyl, phenyl and het²,
het¹ is a 5- to 7-membered heterocyclic group containing one to three hetero-atoms, each independently selected from N, O and S and which is optionally benzo-fused, said heterocyclic group being fully saturated or partially or fully unsaturated, and being optionally substituted by up to three substituents each independently selected from C₁₋₃ alkyl, CF₃, halo, C₁₋₃ alkoxy, CF₃O, OH, NO₂, CN, NR⁴R⁵, COR⁴, CO₂R⁴, CONR⁴R⁵, S(O)ₚ(C₁₋₃ alkyl), CH₂NR⁴R⁵, NR⁴COR⁵, COCF₃, CH₂OH, S(O)ₚCF₃, C(=NH)NH₂, C₂₋₃ alkynyl, C₂₋₃ alkenyl, phenyl and het², and, when R³ is het¹, it is linked to the adjacent O atom by a carbon atom,
het² is a 5- to 7-membered heterocyclic group containing one to three hetero-atoms, which hetero-atoms are each independently selected from N, O and S, which group may be fully saturated or partially or fully unsaturated,
and p is 0, 1 or 2,
and the pharmaceutically acceptable derivatives thereof.

It is to be understood that the term pharmaceutically acceptable derivatives is meant to include veterinarily acceptable derivatives.

Pharmaceutically acceptable derivatives include those compounds in which the functional groups explicitly recited above have been derivatised to provide prodrugs which can be converted to the parent compound *in vivo.* Such prodrugs are discussed in Drugs of Today, Vol. 19, 499-538 (1983) and Annual Reports in Medicinal Chemistry, Vol. 10, Ch. 31 p306-326. In addition, pharmaceutically acceptable derivatives include pharmaceutically acceptable salts, such as alkali metal salts (for example sodium salts) of any acidic groups that may be present. Also included are zwitterionic species which may exist.

Halo means fluoro, chloro, bromo or iodo.

Alkyl, alkenyl and alkynyl groups may be straight chain, branched or cyclic where the number of carbon atoms allows.

Preferably R¹ is either
a) a phenyl group; or
b) a 5-7 membered heterocycle containing 1-3 heteroatoms, each independently selected from O, S and N;
said phenyl and heterocycle being optionally substituted by 1-3 substituents each independently selected from;
i) halo; and
ii) C₁₋₆ alkyl optionally substituted by OH or CO₂H.

More preferably R¹ is a phenyl group optionally substituted by C₁₋₆ alkyl, said alkyl being optionally further substituted by OH or CO₂H.

Most preferably R¹ is phenyl substituted at the 4 position by t-butyl or 2-hydroxy-1,1-dimethylethyl.

Preferably R² is either
a) a phenyl group; or
b) a 5-7 membered heterocycle containing 1-3 heteroatoms, each independently selected from O, S and N and which is optionally benzofused;
said phenyl and heterocycle being optionally substituted by 1-3 substituents selected from:
i) halogen; or
ii) C₁₋₆ alkyl optionally substituted by OH or CO₂H.

More preferably R² is benzodioxol or 4-methylphenyl.

Most preferably R² is a 1,3-benzodiox-5-ol.

Preferably R³ is:
i) hydrogen;
ii) C₁₋₆ alkyl;
iii) C(O)C₁₋₆ alkyl;
iv) phenyl; or
v) a 5-7 membered heterocycle containing 1-3 heteroatoms each independently selected from O, S and N, said heterocycle being optionally benzofused, and
   said phenyl or heterocycle being optionally further substituted by:
   i) halo;
   ii) SOₚR⁴, where p is 0, 1 or 2;
   iii) (C₁₋₅ alkyl)OH;
   iv) (C₁₋₅ alkyl)CO₂H.

More preferably R³ is hydrogen, C(O)CH₃ or pyrimidine optionally substituted by chloro, bromo, SOₚCH₃, where p is 0, 1 or 2, (C₁₋₅ alkyl)OH and (C₁₋₅ alkyl)CO₂H.

Most preferably R³ is 4-chloropyrimidinyl

Preferably R⁴ and R⁵ are hydrogen or C₁₋₆ alkyl.
More preferably R⁴ and R⁵ are hydrogen or C₁₋₃ alkyl.
Most preferably R⁴ and R⁵ are CH₃.

Preferably R⁶ and R⁷ are hydrogen or CH₃.
More preferably R⁶ and R⁷ are hydrogen.

A preferred set of compounds are those described in the Examples and pharmaceutical derivatives thereof.

Most preferred are the compounds:
*N*-(4-(1,3-benzodioxol-5-yl)-3-{2-[(5-chloro-2-pyrimidinyl) oxy]ethoxy}-5-isoxazolyl)-4-(*tert*-butyl)benzenesulfonamide;
*N*-(4-(1,3-benzodioxol-5-yl)-3-{2-[(5-bromo-2-pyrimidinyl) oxy]ethoxy}-5-isoxazolyl)-4-(*tert*-butyl)benzenesulfonamide; or
*N*-(4-(1,3-benzodioxol-5-yl)-3-{2-[(5-bromo-2-pyrimidinyl)oxy]ethoxy}-5-isoxazolyl)-4-(2-hydroxy-1,1-dimethylethyl)benzenesulfonamide.

The substances of the invention may possess one or more chiral centres and so exist in a number of stereoisomeric forms. All stereoisomers and mixtures thereof are included in the scope of the present invention. Racemic substances may either be separated using preparative HPLC and a column with a chiral stationary phase or resolved to yield individual enantiomers utilising methods known to those skilled in the art. In addition, chiral intermediates may be resolved and used to prepare chiral compounds of formulae (IA) and (IB).

The substances of the invention are useful because they blockade ET receptors and are thus useful in the treatment or prevention of any diseases for which such a blockade is beneficial. More particularly, they are useful in the treatment and prevention of restenosis, acute/chronic renal failure, hypertension including pulmonary and systemic hypertension; benign prostatic hypertrophy, male erectile dysfunction, prostate cancer, metastatic bone cancer, congestive heart failure, stroke, subarachnoid haemorrhage, angina, atherosclerosis, cerebral and cardiac ischaemia, prevention of ischaemia/reperfusion injury (e.g. allografts), cyclosporin induced nephrotoxicity, glaucoma, radiocontrast nephropathy, diabetic neuropathy, allergy, restoration of organ perfusion in haemorrhagic shock, lipoprotein lipase related disorders, chronic obstructive pulmonary disease and hyaline membrane disease in newborn. The treatment of congestive heart failure, restenosis, renal failure and systemic and pulmonary hypertension are of particular interest. The substances of the invention may be administered alone or as part of a combination therapy.

The invention further provides methods for the production of the compounds of the invention, which are described below and in the Examples and Preparations section. The skilled man will appreciate that the substances of the invention could be made by methods other than those herein described, by adaptation of the methods herein described and/or adaptation of a plethora of methods known in the art. It is to be understood that the synthetic transformation methods specifically mentioned herein may be carried out in various different sequences in order that the desired substances can be efficiently assembled. The skilled chemist will exercise his judgement and skill as to the most efficient sequence of reactions for synthesis of a given target substance.

It will be apparent to those skilled in the art that sensitive functional groups may need to be protected and deprotected during synthesis of a substance of the invention. This may be achieved by conventional techniques, for example as described in 'Protective Groups in Organic Synthesis' by T W Greene and P G M Wuts, John Wiley and Sons Inc, 1991.

In the Methods below, unless otherwise specified, the substituents are as defined above with reference to the compounds of formula (I) above.

### Method 1

Compounds of formula (I) can be made via reaction of the corresponding compound of formula (II) as appropriate, 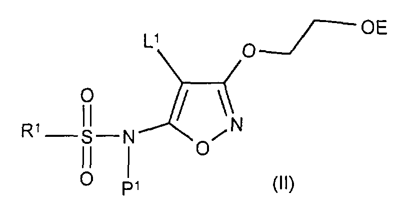 where L¹ is a leaving group such as Cl, Br, I or triflate, P¹ is H, R¹SO₂ or a nitrogen-protecting group such as methoxymethyl, iso-butoxycarbonyl, etc., and E is R³ as defined with reference to compounds of formula (I) above, or E is a suitable oxygen-protecting group such as (C₁₋₄ alkyl)CO, with a reagent which is equivalent to R²-Y. For instance the reagent R²-Y can be an organometallic species such as an arylboronic acid R²-B(OH)₂, aryltin species R²-SnBu₃ or an arylzinc species R²-ZnCl. Such reagent types are well known in the art as, are the reaction conditions, catalysts, co-reagents, solvents, etc. Particularly preferred are those reactions where (II) and R²-Y are coupled using a palladium or other suitable transition metal coupling reaction.

This type of reaction is exemplified in Preparation 6. Compounds of formula (II) may be made via conventional methods as exemplified in Preparation 5.

Where E is a suitable oxygen-protecting group such as (C₁₋₄ alkyl)CO, the protecting group may be removed during the reaction of method 1, using appropriate conditions, or subsequently, as discussed below.

### Method 2

Compounds of formula (I) where R³ is hydrogen may be made via hydrolysis of the corresponding ester of formula (III): 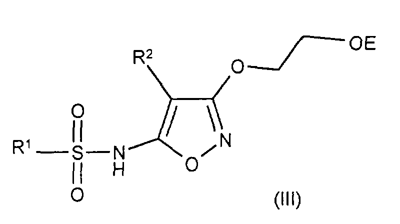 wherein E is (C₁₋₄ alkyl)CO, for example by treatment with aqueous base such as aqueous sodium hydroxide or aqueous potassium carbonate, in a suitable solvent such as methanol or ethanol.

Compounds of formula (III) may be made by the process of method 1, wherein E is (C₁₋₄ alkyl)CO, and is not removed during method 1.

### Method 3

Compounds of formula (I) where R³ is aryl¹ or het¹ can be made from the corresponding compound of formula (I) where R³ is H, for instance via reaction of the compound of formula (I) where R³ is H with a reagent of formula "R³-L²", where "L²⁻" is a suitable leaving group such as a halo, arenesulphonate, C₁₋₄ alkanesulphonate or perfluoro(C₁₋₄ alkane)sulphonate ion, suitably a chloride, phenylsulphonate, p-toluenesulphonate or mesylate ion, suitably in the presence of a base such as sodium hydride or potassium carbonate, in a suitable inert organic solvent such as N,N-dimethylformamide (DMF) or tetrahydrofuran (THF). Reagents of the formula "R³-L²" are well known in the art as, are the reaction conditions, catalysts, co-reagents, solvents, etc.

Preferably, R³ is 5-chloro-pyrimidin-2-yl or 5-bromo-pyrimidin-2-yl.

Compounds of formula (I) where R³ is H, may be made via conventional methods as exemplified in Preparation 6.

This type of reaction is mentioned in for example US Patent 5,728,706 and Tetrahedron (1984) **40,** 1433, and is exemplified below in Examples 2, 3 & 4.

Where desired or necessary the compound of formula (I) is converted into a pharmaceutically acceptable salt thereof. A pharmaceutically acceptable salt of a compound of formula (I) may be conveniently be prepared by mixing together solutions of a compound of formula (I) and the desired acid or base, as appropriate. The salt may be precipitated from solution and collected by filtration, or may be collected by other means such as by evaporation of the solvent, or by other means known to a man skilled in the art.

The compounds of the invention may be separated and purified by conventional methods.

The invention also provides a compound of formula (I) or a pharmaceutically acceptable derivative thereof for use as medicament.

The invention also provides for the use of a compound of formula (I) or pharmaceutically acceptable derivative thereof as defined above, in the manufacture of a medicament for the treatment of a condition mediated by endothelin, particularly endothelin ET_{A}.

The invention also provides for the use of a compound of formula (I) or pharmaceutically acceptable derivative thereof as defined above, in the manufacture of a medicament for the treatment of restenosis, acute/chronic renal failure, pulmonary hypertension, systemic hypertension; benign prostatic hyperplasia, male erectile dysfunction, prostate cancer, metastatic bone cancer, congestive heart failure, stroke, subarachnoid haemorrhage, angina, atherosclerosis, cerebral and cardiac ischaemia, prevention of ischaemia/reperfusion injury (e.g. allografts), cyclosporin induced nephrotoxicity, glaucoma, radiocontrast nephropathy, diabetic neuropathy, allergy, restoration of organ perfusion in haemorrhagic shock, lipoprotein lipase related disorders, chronic obstructive pulmonary disease and hyaline membrane disease in newborn.

The invention also provides a method of treating conditions mediated by endothelin, particularly ET_{A}, which comprises administering to a patient in need of such treatment a therapeutically effective amount of a compound of formula (I) or a pharmaceutically acceptable derivative thereof.

Particularly suitable conditions are selected from: restenosis, acute/chronic renal failure, pulmonary hypertension, systemic hypertension; benign prostatic hyperplasia, male erectile dysfunction, prostate cancer, metastatic bone cancer, congestive heart failure, stroke, subarachnoid haemorrhage, angina, atherosclerosis, cerebral and cardiac ischaemia, prevention of ischaemia/reperfusion injury (e.g. allografts), cyclosporin induced nephrotoxicity, glaucoma, radiocontrast nephropathy, diabetic neuropathy, allergy, restoration of organ perfusion in haemorrhagic shock, lipoprotein lipase related disorders, chronic obstructive pulmonary disease and hyaline membrane disease in newborn.

Reference to treatment herein includes prevention of undesirable conditions as well as alleviation or cure of said conditions.

The biological activity of the substances of the invention may be demonstrated as follows:

### Dog Binding assay

Competition between test substances and ligands binding to canine endothelin receptors is determined as follows:

### Dog ET_{A} Binding Assay

50µl of a 500pM solution of ¹²⁵I-PD-151242 (Specific activity 2,000 Ci/mM) is mixed with 50µl samples of test substances (final concentrations in the range from 0.01-10,000nM). 100µg of purified dog kidney homogenate is added in 150µl of the following buffer: 50mM Tris, 10mM MgCl₂ and 0.05% Bovine Serum Albumen at pH 7.4. The solution is incubated at room temperature for 2 hours. After the incubation, the unbound ligand is separated from receptor bound ligand by filtration with a Brandel cell harvester, followed by 5 washes with buffer (Tris 50mM, MgCl₂ 10mM). Filter papers are counted for radioactivity and the Kᵢ (an IC₅₀ corrected for the dissociation constant and concentration of the ligand added) is determined for the concentration range tested.

### Dog ET_{B} Binding Assay

50µl of a 100pM solution of ¹²⁵I-IRL-1620 (Specific activity 2,200 Ci/mM) is mixed with 50µl samples of test substances (final concentrations in the range from 0.01-10,000nM). 50µg of purified Dog cerebellum homogenate is added in 150µl of the following buffer; 50mM Tris, 10mM MgCl₂ and 0.05% Bovine Serum Albumen at pH 7.4. The solution is incubated at 30°C for 90 minutes. After the incubation, the unbound ligand is separated from receptor bound ligand by filtration with a Brandel cell harvester, followed by 5 washes with buffer (Tris 50mM, MgCl₂ 10mM). Filter papers are counted for radioactivity and the Kᵢ (an IC₅₀ corrected for the dissociation constant and concentration of the ligand added) is determined for the concentration range tested.

The compounds of the present invention were investigated using the above assay and demonstrated strong ET_{A} affinity and a marked selectivity for the ET_{A} over the ET_{B} receptor.

The substances of the invention can be administered alone but will generally be administered in admixture with a pharmaceutical carrier selected with regard to the intended route of administration and standard pharmaceutical or veterinary practice.

For example they can be administered orally in the form of tablets containing such excipients as starch or lactose or in capsules or ovules either alone or in admixture with excipients or in the form of elixirs, solutions or suspensions containing the substance in a liquid carrier, for example a vegetable oil, glycerine or water with a flavouring or colouring agent. They can be injected parenterally, for example intravenously, intramuscularly or subcutaneously. For parental administration, they are best used as sterile aqueous solutions which may contain other substances, for example, enough glucose or salts to make the solution isotonic with blood. For parenteral administration the substance may also be administered as a solution or suspension in a suitable oil, for example polyethylene glycol, lecithin or sesame oil.

Substances of the invention may also be administered through inhalation of a solution, suspension or emulsion that may be administered as a dry powder or in the form of an aerosol using a conventional propellant such as dichlorodifluoromethane.

For oral or parenteral administration to human patients the daily dosage levels of substances of the invention will be from 0.01 to 30 mg/kg (in single or divided doses) and preferably will be in the range 0.01 to 5 mg/kg. Thus tablets will contain 1mg to 0.4g of substance for administration singly or two or more at a time, as appropriate. The above dosages are, of course only exemplary of the average case and there may be instances where higher or lower doses are merited, and such are within the scope of the invention.

Alternatively the substances of the invention can be administered in the form of a suppository or pessary, or they may be applied topically in the form of a lotion, solution, cream, ointment or dusting powder or in the form of a medicated plaster, patch or membrane. For example they may be incorporated in a cream containing an aqueous emulsion of polyethylene glycols or liquid paraffin. The compounds may also be administered intranasally.

For veterinary use although it is possible to administer a substance of the invention directly without any formulation, the substances are preferably employed in the form of a pharmaceutical or veterinary formulation comprising a pharmaceutically or veterinarily acceptable carrier, diluent or excipient and a substance of the invention.

Such compositions will contain from 0.1 percent by weight to 90.0 percent by weight of the active ingredient.

The methods by which the compounds may be administered include oral administration by capsule, bolus, tablet or drench, topical administration as an ointment, a pour-on, spot-on, dip, spray, mousse, shampoo or powder formulation or, alternatively, they can be administered by injection (e.g. subcutaneously, intramuscularly or intravenously), or as an implant.

Such formulations are prepared in a conventional manner in accordance with standard veterinary practice. Thus capsules, boluses or tablets may be prepared by mixing the active ingredient with a suitable finely divided diluent or carrier additionally containing a disintegrating agent and/or binder such as starch, lactose, talc or magnesium stearate, etc. Oral drenches are prepared by dissolving or suspending the active ingredient in a suitable medium. Pour-on or spot-on formulations may be prepared by dissolving the active ingredient in an acceptable liquid carrier vehicle such as butyl digol, liquid paraffin or a non-volatile ester, optionally with the addition of a volatile component such as propan-2-ol.

Alternatively, pour-on, spot-on or spray formulations can be prepared by encapsulation, to leave a residue of active agent on the surface of the animal. Injectable formulations may be prepared in the form of a sterile solution which may contain other substances, for example enough salts or glucose to make the solution isotonic with blood. Acceptable liquid carriers include vegetable oils such as sesame oil, glycerides such as triacetin, esters such as benzyl benzoate, isopropyl myristrate and fatty acid derivatives of propylene glycol, as well as organic solvents such as pyrrolidin-2-one and glycerol formal. The formulations are prepared by dissolving or suspending the active ingredient in the liquid carrier such that the final formulation contains from 0.1 to 10% by weight of the active ingredient.

These formulations will vary with regard to the weight of active substance contained therein, depending on the species of animal to be treated, the severity and type of infection and the body weight of the animal. For parenteral, topical and oral administration, typical dose ranges of the active ingredient are 0.01 to 100 mg per kg of body weight of the animal. Preferably the range is 0.1 to 10 mg per kg.

The compositions are preferably formulated in a unit dosage form, each dosage containing from about 1 to about 500 mg, more usually about 5 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

As an alternative for veterinary use the substances may be administered with animal feedstuff and for this purpose a concentrated feed additive or premix may be prepared for mixing with the normal animal feed.

Thus, according to a further aspect of the invention, there are provided pharmaceutical formulations comprising a substance of the invention, as defined above, and a pharmaceutically-acceptable adjuvant, diluent or carrier.

High performance liquid chromatography (HPLC) retention times and UV spectra were recorded using a Hewlett-Packard 1090 LUSI diode-array spectrophotometer (method A). All NMR spectra were measured in CDCl₃ or MeOD by an Inova 300 MHz or 400 MHz spectrometer unless otherwise indicated and peak positions are expressed in parts per million (ppm) downfield from tetramethylsilane. The peak shapes are denoted as follows: s, singlet; d, doublet; t, triplet; q, quartet; m, multiplet; br broad. High resolution MS data was acquired on a AutoSpecQ with electrospray ionisation (ESI) or thermospray ionisation (TSPI) using a PEG reference (or on a Bruker Apex II FTMS with ESI where indicated).

| MY Inoculum and Production Medium | |
|---|---|
| Glucose | 10g |
| Peptone (Difco™) | 5g |
| Yeast extract (Oxoid™) | 3g |
| Malt extract (Oxoid™) | 5g |
| Tap water | 1l |
| NaOH | To pH 6.3 - 6.5 |

### Example 1:

### N-(4-(1,3-benzodioxol-5-yl)-3-{2-[(5-bromo-2-pyrimidinyl)oxy]ethoxy}-5-isoxazolyl)-4-(2-hydroxy-1,1-dimethylethyl)benzenesulfonamide

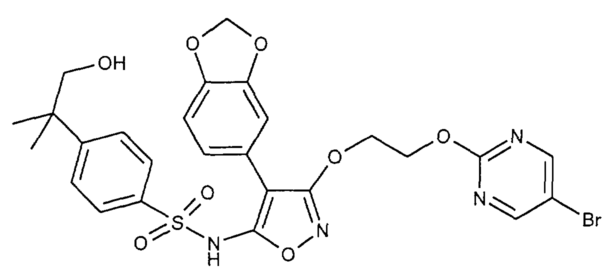

*Amycolata autotrophica* ATCC35203 maintained on a quarter strength ATCC172 agar slope was inoculated as a loopful of spores into a 300 ml Erlenmeyer flasks each containing 50ml of of MY inoculum medium. This was allowed to incubate for 2 days at 28°C, 200rpm on an Infors Multitron™ Shaker with 1" throw. Two mls of this inoculum was then transferred to each of twenty 300ml Erlenmeyer flask containing 50ml of MY production medium and incubated under the same conditions for a further 24 hours. At this point 5mg of *N*-(4-(1,3-benzodioxol-5-yl)-3-{2-[(5-bromo-2-pyrimidinyl)oxy]ethoxy}-5-isoxazolyl)-4-(*tert*-butyl)benzenesulfonamide **(Example 2)** dissolved in 0.5ml of methanol was added to each of the flasks and the fermentation allowed to continue under the same conditions for a further 144 hours. Each flask was then extracted with 200mls of ethyl acetate and the combined ethyl acetate solubles concentrated to dryness to give a gum solid.

The crude extract was purified by preparative reversed phase HPLC with a Phenomenex Magellen™ 5µ C18 column (150mm x 21.2mm) in two injections. Using a gradient mobile phase of 35:65 to 20:80 water/methanol from 1.5 to 29 minutes at a flow rate of 20ml/min, the product was eluted at 4.1 minutes. The product fractions were purified again on the same column in one injection. Using a gradient mobile phase of 85% to 15% water/methanol from 0 to 25 minutes at a flow rate of 20ml/min, the product was eluted at 14.9 minutes. The product fractions were concentrated under reduced pressure to yield the title compound as a colourless amorphous solid (2.0 mg).
δH (300MHz, CDCl₃) 8.40 (2H, s), 7.70 (2H, d), 7.10 (2H, d), 7.05 (2H, m), 6.55 (1H, d), 5.75 (2H, s), 4.55 (2H, m), 4.40 (2H, m), 3.35 (2H, s), 1.05 (6H, s)
m/z (ESI) [M+H]⁺ = 633.0655, C₂₆H₂₆⁷⁹BrN₄O₈S requires 633.0655
m/z (ESI) [M+Na]⁺ = 655.0481, C₂₆H₂₅⁷⁹BrN₄O₈SNa requires 655.0474

### Example 2:

### N-(4-(1,3-benzodioxol-5-yl)-3-{2-[(5-bromo-2-pyrimidinyl) oxy]ethoxy}-5-isoxazolyl)-4-(tert-butyl)benzenesulfonamide

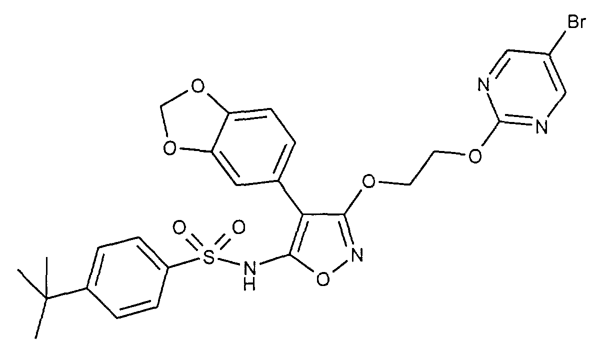

To a solution of *N*-[4-(1,3-benzodioxol-5-yl)-3-(2-hydroxyethoxy)-5-isoxazolyl]-4-(*tert*-butyl)benzenesulfonamide **(Preparation 6)** (90mg) in tetrahydrofuran (1.5ml), purged with nitrogen three times, was added sodium hydride (16mg of a 60% dispersion in oil) and the reaction mixture stirred for 15 minutes. After which time a solution of 5-bromo-2-chloropyrimidine (41mg) in tetrahydrofuran (0.5ml) was added to the reaction followed by dimethylacetamide (0.1ml). The reaction mixture was left stirring at room temperature overnight. This solution was added to a stirring mixture of ether (30ml) and citric acid (1.0M, 30ml) The organics were separated and further washed with brine (30ml) and dried over magnesium sulfate before being concentrated in-vacuo to yield the crude material (90mg). This was purified by HPLC on a 5µ ODS Phenomenex Magellen™ column with a isocratic elution of 0.1M NH₄OAc (55%) and acetonitrile (45%) to yield the desired product as a white solid (10mg).
δH (300MHz, CDCl₃) 8.50(2H, s), 7.85(2H, d), 7.50(2H, d), 6.85(2H, d), 6.75(1H, d), 5.95(2H, s), 4.75(2H, m), 4.65(2H, m), 1.35(9H, s)
^{*m*}/_{*z*} (thermospray) [MH⁺] = 617.5, C₂₆H₂₆BrN₄O₇S requires 617.5.

### Example 3:

### N-(4-(1,3-benzodioxol-5-yl)-3-{2-[(5-chloro-2-pyrimidinyl) oxy]ethoxy}-5-isoxazolyl)-4-(tert-butyl)benzenesulfonamide

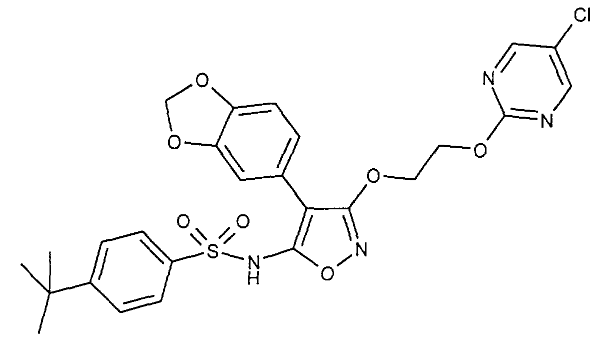

To a solution of *N*-[4-(1,3-benzodioxol-5-yl)-3-(2-hydroxyethoxy)-5-isoxazolyl]-4-(*tert*-butyl)benzenesulfonamide **(Preparation 6)** (104mg) in tetrahydrofuran (3ml), purged with nitrogen three times, was added sodium hydride (19mg of a 60% dispersion in oil) and the reaction mixture stirred for 15 minutes. After which time a solution of 5-chloro-2-(methylsulfonyl)pyrimidine (48mg) in dimethylformamide (0.5ml) was added to the reaction mixture and then left stirring at room temperature overnight. The reaction mixture was quenched with citric acid (1.0M, 20ml) and extracted into ethyl acetate (30ml). The organics were washed with water (20ml), brine (20ml) and dried over magnesium sulfate before being concentrated *in vacuo* to yield the crude material (45mg). This was purified by HPLC on a 5µ ODS Phenomenex Magellen™ column with a gradient elution of 0.1M NH₄OAc (95% to 55%) and acetonitrile (5% to 45%) to yield the desired product as a white solid (25mg).
δH (300MHz, CDCl₃) 8.40(2H, s), 7.80(2H, d), 7.45(2H, d), 6.85(2H, d), 6.70(1H, d), 5.95(2H, s), 4.70(2H, m), 4.60(2H, m), 1.35(9H, s)
6.75(1H, d), 5.95(2H, s), 4.75(2H, m), 4.65(2H, m), 1.35(9H, s)
^{*m*}/_{*z*} (thermospray) [MH⁺] = 573.1, C₂₆H₂₆ClN₄O₇S requires 573.0.

### Example 4:

### N-[4-(1,3-benzodioxol-5-yl)-3-(2-{[5-(methylsulfanyl)-2-pyrimidinyl] oxy}ethoxy)-5-isoxazolyl]-4-(tert-butyl)benzenesulfonamide

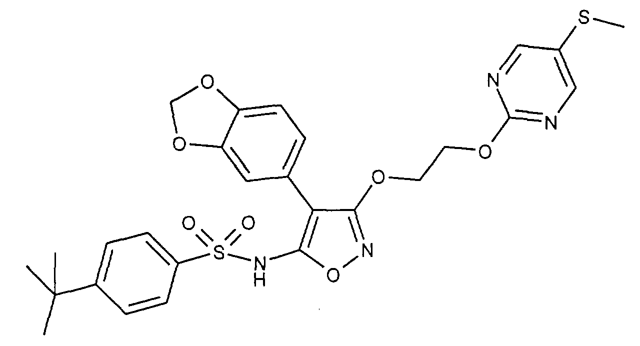

To a solution of *N*-[4-(1,3-benzodioxol-5-yl)-3-(2-hydroxyethoxy)-5-isoxazolyl]-4-(*tert*-butyl)benzenesulfonamide **(Preparation 6)** (109mg) in tetrahydrofuran (3ml), purged with nitrogen three times, was added sodium hydride (20mg of a 60% dispersion in oil) and the reaction mixture stirred for 15 minutes. After which time a solution of 2-chloro-5-(methylsulfanyl)pyrimidine (42mg) in dimethylformamide (0.5ml) was added to the reaction mixture and then left stirring at room temperature overnight. The reaction mixture was quenched with citric acid (1.0M, 10ml) and extracted into ethyl acetate (15ml). The organics were washed with water (10ml), brine (10ml) and dried over magnesium sulfate before being concentrated *in vacuo* to yield the crude material (150mg). This was purified by HPLC on a 5µ ODS Phenomenex Magellen™ column with a gradient elution of 0.1M NH₄OAc (95% to 50%) and acetonitrile (5% to 50%) to yield the desired product as a white solid (16mg).
δH (300MHz, CDCl₃) 8.50(2H, s), 7.80(2H, d), 7.60(1H, br), 7.50(2H, d), 6.80(2H, d), 6.65(1H, d), 5.95(2H, s), 4.70(2H, m), 4.60(2H, m), 2.45(3H, s), 1.35(9H, s)
^{*m*}/_{*z*} (thermospray) [MH⁺] = 584.7, C₂₇H₂₉N₄O₇S₂ requires 584.7.

### Example 5:

### N-[4-(1,3-benzodioxol-5-yl)-3-(2-{[5-(methylsulfonyl)-2-pyrimidinyl] oxy}ethoxy)-5-isoxazolyl]-4-(tert-butyl)benzenesulfonamide

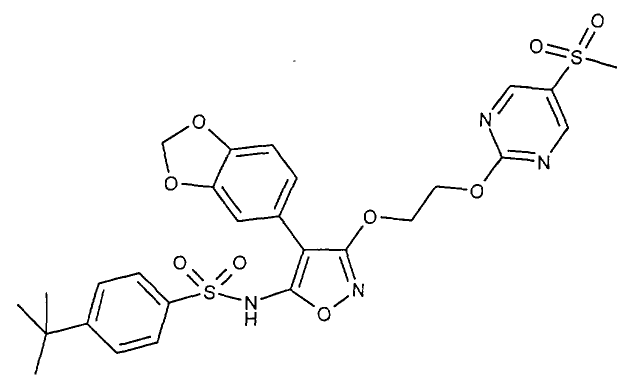

To a suspension of *N*-[4-(1,3-benzodioxol-5-yl)-3-(2-{[5-(methyl sulfanyl)-2-pyrimidinyl] oxy}ethoxy)-5-isoxazolyl]-4-(*tert*-butyl) benzenesulfonamide **(Example 4)** (15mg) in dichloromethane (0.3ml) was added m-chloroperoxybenzoic acid (7mg) in dichloromethane (0.2ml). The reaction mixture was left stirring at room temperature overnight. The solvent was removed *in vacuo* to yield the crude material as an orange solid (20mg). This was purified by HPLC on a 5µ ODS Phenomenex Magellen™ column with a gradient elution of 0.1M NH₄OAc (95% to 60%) and acetonitrile (5% to 40%) to yield the desired product as a white solid (6mg).
δH (300MHz, CDCl₃) 8.95(2H, s), 7.80(2H, d), 7.50(2H, d), 7.10(1H, br), 6.80(2H, d), 6.70(1H, d), 5.95(2H, s), 4.90(2H, m), 4.70(2H, m), 3.15(3H, s), 1.35(9H, s)
^{*m*}/_{*z*} (thermospray) [MH⁺] = 616.6, C₂₇H₂₉N₄O₉S₂ requires 616.7

### Preparation 1:

### tert-Butyl 3-[2-(acetoxy)ethoxy]-5-amino-4-isoxazolecarboxylate

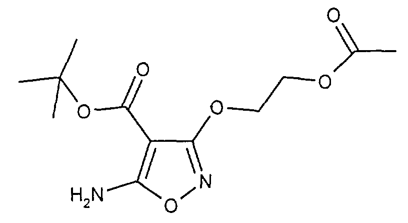

To a stirring solution of *tert*-butyl 5-amino-3-(2-hydroxyethoxy)-4-isoxazolecarboxylate ( R. Neidlein, *J. Heterocyclic Chem*., 1989, **26,** 1335) (5.60g) and triethylamine (3.36ml) in tetrahydrofuran (50ml) at room temperature was added 4-dimethylaminopyridine (280mg) followed by acetic anhydride (2.81g). The reaction was stirred for 2 hours at room temperature. The solvent was removed *in vacuo* to yield the crude material (6.0g). The crude material was purified using the Biotage™ Flash 40i System (silica, 90g), eluting with ethyl acetate:hexane (1:1) to yield the product as an off white solid (5.0g)
δH (300MHz, CDCl₃) 5.80 (2H, br), 4.40-4.45 (4H, m), 2.10 (3H, s), 1.55 (9H, s)

### Preparation 2:

### tert-Butyl 3-[2-(acetoxy)ethoxy]-5-({[4-(tert-butyl)phenyl]sulfonyl}amino)-4-isoxazolecarboxylate

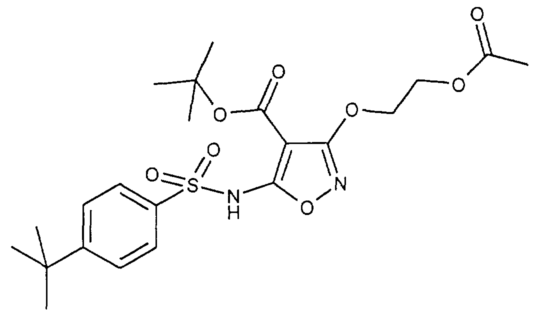

To a stirring solution of tert-butyl 3-[2-(acetoxy)ethoxy]-5-amino-4-isoxazolecarboxylate **(Preparation 1)** (6.0g) in tetrahydrofuran (55ml) under an atmosphere of nitrogen, was added sodium hydride (1.68g of a 60% dispersion in oil). The reaction was stirred for 15 minutes after which time *tert* butylbenzenesulfonyl chloride (5.14g) was added. The reaction was stirred at room temperature overnight. The solvent was removed *in vacuo* and redissolved in dichloromethane (50ml) and then washed with water (50ml with 3 drops of HCl). The organics were further washed with brine (40ml), dried over magnesium sulfate and concentrated under reduced pressure to yield the crude material (7.0g). The crude material was purified using the Biotage™ Flash 40i system (silica, 90g) with a gradient elution of ethyl acetate (10% to 95%) and dichloromethane (90% to 5%) to yield the desired product as a white solid (3.5g).
δH (300MHz, *d*_{*6*}DMSO) 7.75 (2H, d), 7.45 (2H, d), 4.15 - 4.30 (4H, m), 2.00 (3H, s), 1.40 (9H, s), 1.30 (9H, s)

### Preparation 3:

### 2-{[5-({[4-(tert-butyl)phenyl]sulfonyl}amino)-3-isoxazolyl]oxy}ethyl acetate

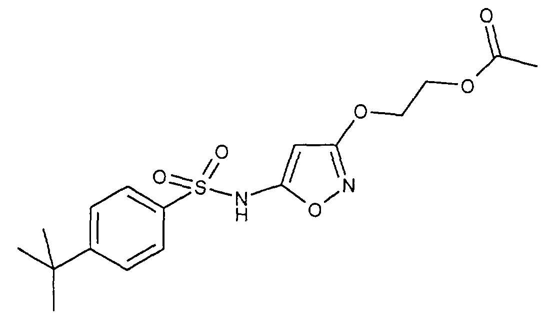

To a solution of *tert*-butyl 3-[2-(acetoxy)ethoxy]-5-({[4-(tert-butyl)phenyl]sulfonyl}amino)-4-isoxazolecarboxylate **(Preparation 2)** (3.16g) in dichloromethane (40ml) was added trifluoroacetic acid (20ml). The reaction mixture was heated to reflux for 3 hours. After which time the reaction mixture was basified to pH8 using sodium hydrogen carbonate solution and then re-acidified to pH2 using aqueous hydrochloric acid (1.0M). This aqueous layer was extracted into ethyl acetate (2 x 250ml) and the organics combined, dried over magnesium sulfate and the solvent removed *in vacuo* to yield the crude material (2.6g). This was dissolved up in toluene and refluxed for 2 hours. The solvent was removed *in vacuo* to yield the crude material as a light brown solid (2.3g). The crude material was purified using the Biotage™ Flash 40i system (silica, 90g) and eluted with hexane:ethyl acetate (5:2) to yield the product as a white solid (1.2g)
δH (300MHz, CDCl₃) 7.80 (2H, d), 7.55 (2H, d), 5.65 (1H, s), 4.35 - 4.40 (4H, m), 2.10 (3H, s), 1.35 (9H, s).

### Preparation 4:

### 2-{[5-({[4-(tert-butyl)phenyl]sulfonyl}amino)-4-iodo-3-isoxazolyl]oxy}ethyl acetate

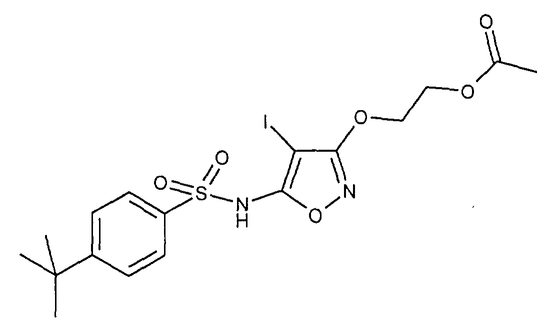

To a stirring solution of 2-{[5-({[4-(*tert*-butyl)phenyl]sulfonyl}amino)-3-isoxazolyl]oxy}ethyl acetate **(Preparation 3)** (1.17g) in tetrahydrofuran (10ml) was added N-iodosuccinimide (0.76g). The reaction mixture was left stirring at room temperature overnight. The solvent was removed *in vacuo* to yield the crude material as a brown oil (1.5g). The crude material was purified using the Biotage™ Flash 40i system (silica, 90g) and eluted with hexane:ethyl acetate (1:9) to yield the product as a brown oil.
δH (300MHz, CDCl₃) 7.90 (2H, d), 7.55 (2H, d), 4.35-4.45 (4H, m), 2.10 (3H, s), 1.35 (9H, s).

### Preparation 5:

### 2-({5-[{[4-(tert-butyl)phenyl]sulfonyl}(isobutoxycarbonyl) amino]-4-iodo-3-isoxazolyl}oxy)ethyl acetate

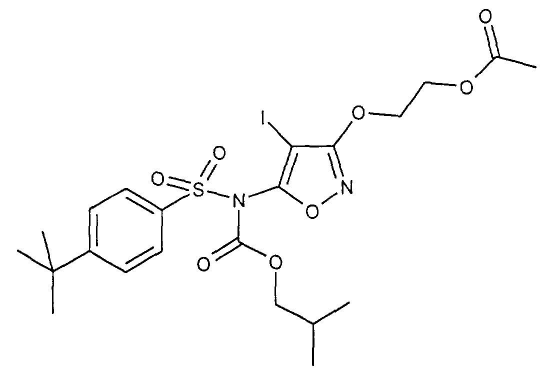

To a stirring solution of 2-{[5-({[4-(*tert*-butyl)phenyl]sulfonyl}amino)-4-iodo-3-isoxazolyl]oxy}ethyl acetate **(Preparation 4)** (9.44g) in dichloromethane was added pyridine (1.65ml) followed by the slow addition of isobutylchloroforomate (2.41ml) over 10 minutes. The reaction mixture was stirred at room temperature for one hour. The solvent was removed *in vacuo* to yield the crude material. This was purified using column chromatography (300g silica, compound loaded with dichloromethane (15ml) using a gradient elution of hexane (100% to 75%) and ethyl acetate (0% to 25%) to yield the desired compound as a yellow oil (7.70g)
δH (300MHz, CDCl₃) 8.05 (2H, d), 7.60 (2H, d), 4.50 (2H, m), 4.45 (2H, m), 3.90 (2H, d), 2.15 (3H, s), 1.85 (1H, m), 1.35 (9H, s), 0.80 (6H, d).

### Preparation 6:

### N-[4-(1,3-benzodioxol-5-yl)-3-(2-hydroxyethoxy)-5-isoxazolyl]-4-(tert-butyl)benzenesulfonamide

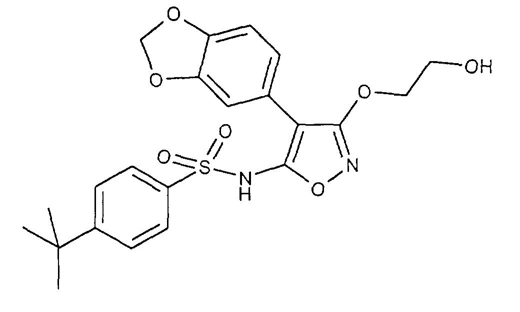

To a stirring solution of 2-({5-[{[4-(*tert*-butyl)phenyl]sulfonyl} (isobutoxycarbonyl)amino]-4-iodo-3-isoxazolyl}oxy)ethyl acetate **(Preparation 5)** (2.41g) in dioxane (25ml) was added 3,4-methylenedioxybenzeneboronic acid (0.72g) followed by cesium carbonate (5.15g) and water (3ml). The reaction mixture was purged with nitrogen three times, after which time tetrakis(triphenylphosphine)-palladium(0) (140mg). The reaction mixture was heated to reflux for about 2 hours. Ethanol (50ml) and sodium hydroxide (2M, 50ml) were added to the reaction mixture and then this was stirred at room temperature overnight. The reaction mixture was concentrated *in vacuo* and the solid residue was partitioned between saturated ammonium chloride solution (100ml) and ethyl acetate (100ml). The organics were washed with brine (50ml), dried over sodium sulfate and concentrated under reduced pressure to yield the crude material as a brown oil. The crude material was purified by column chromatography (100g silica) using a gradient elution of hexane (50% to 0%) and ethyl acetate (50% to 100%) and also with methanol (5% in ethyl acetate) to yield the product as a light brown foam (770mg).
δH (300MHz, *d*_{*6*}DMSO) 7.65 (2H, d), 7.50 (2H, d), 6.95 (1H, s), 6.90 (2H, d), 6.80 (2H, d), 6.00 (2H, s), 4.10 (2H, t), 3.70 (2H, t), 1.15 (9H, s).

## Claims

1. A compound of formula (I) 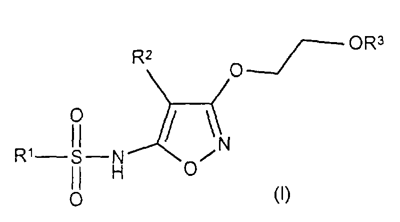 wherein
R¹ is:
a) a phenyl group; or
b) an optionally benzo-fused 5- or 6-membered heterocyclic group with one to three heteroatoms in the heterocyclic ring, which heteroatoms are independently selected from N, O and S; or
c) CHR⁶CHR⁷Ph, CR⁶:CR⁷Ph;
each of which groups (a) (b) and (c) is optionally substituted by up to three substituents each independently selected from:
i) halo;
ii) C₁₋₆ alkyl optionally substituted by OH, halogen, NR⁴R⁵ OCOR⁴ or CO₂R⁴;
iii) CN;
iv) O(C₁₋₆ alkyl optionally substituted by one or more halogen); and
v) CO₂R⁴;
R⁴ and R⁵ are each independently H or C₁₋₆ alkyl optionally substituted by one or more halo,
R⁶ and R⁷ are each independently H or C₁₋₃ alkyl,
R² is aryl¹ or het¹,
R³ is H, C₁₋₆ alkyl, C(O)R⁴, CONHaryl¹, CONHhet¹, aryl¹ and het¹,
Wherein aryl¹ is a phenyl or naphthyl group optionally substituted by up to three substituents each independently selected from C₁₋₃ alkyl, CF₃, halo, C₁₋₃ alkoxy, OCF₃, OH, NO₂, CN, NR⁴R⁵, COR⁴, CO₂R⁴, CONR⁴R⁵, S(O)ₚ(C₁₋₃ alkyl), CH₂NR⁴R⁵, NR⁴COR⁵, COCF₃, CH₂OH, S(O)ₚCF₃, C(=NH)NH₂, C₂₋₃ alkynyl, C₂₋₃ alkenyl, phenyl and het²,
Wherein het¹ is a 5- to 7-membered heterocyclic group containing one to three hetero-atoms, each independently selected from N, O and S and which is optionally benzo-fused, said heterocyclic group being fully saturated or partially or fully unsaturated, and being optionally substituted by up to three substituents each independently selected from C₁₋₃ alkyl, CF₃, halo, C₁₋₃ alkoxy, CF₃O, OH, NO₂, CN, NR⁴R⁵, COR⁴, CO₂R⁴, CONR⁴R⁵, S(O)ₚ(C₁₋₃ alkyl), CH₂NR⁴R⁵, NR⁴COR⁵, COCF₃, CH₂OH, S(O)ₚCF₃, C(=NH)NH₂, C₂₋₃ alkynyl, C₂₋₃ alkenyl, phenyl and het², and, when R³ is het¹, it is linked to the adjacent O atom by a carbon atom,
wherein het² is a 5- to 7-membered heterocyclic group containing one to three hetero-atoms, which hetero-atoms are each independently selected from N, O and S,
which group may be fully saturated or partially or fully unsaturated,
and p is 0, 1 or 2,
and the pharmaceutically acceptable derivatives thereof.

2. A compound of formula (I) as claimed in claim 1 wherein R¹ is selected from:
a) a phenyl group; or
b) a 5-7 membered heterocycle containing 1-3 heteroatoms, each independently selected from O, S and N; said phenyl and heterocycle being optionally substituted by 1-3 substituents each independently selected from;
i) halo; and
ii) C₁₋₆ alkyl optionally substituted by OH or CO₂H.

3. A compound of formula (I) as claimed in claims 1 and 2 where R¹ is a phenyl group optionally substituted by C₁₋₆ alkyl, said alkyl being optionally further substituted by OH or CO₂H.

4. A compound of formula (I) as claimed in claims 1-3 where R¹ is phenyl substituted at the 4 position by t-butyl or 2-hydroxy-1,1-dimethylethyl.

5. A compound of formula (I) as claimed in claims 1-4 where R² is selected from:
a) a phenyl group; or
b) a 5-7 membered heterocycle containing 1-3 heteroatoms, each independently selected from O, S and N and which is optionally benzofused; said phenyl and heterocycle being optionally substituted by 1-3 substituents selected from:
i) halogen; or
ii) C₁₋₆ alkyl optionally substituted by OH or CO₂H.

6. A compound of formula (I) as claimed in claims 1-5 where R² is benzodioxol or 4-methylphenyl.

7. A compound of formula (I) as claimed in claims 1-6 where R² is a 1,3-benzodiox-5-ol.

8. A compound of formula (I) as claimed in claims 1-7 where R³ is selected from:
i) hydrogen;
ii) C₁₋₆ alkyl;
iii) C(O)C₁₋₆ alkyl;
iv) phenyl; or
v) 5-7 membered heterocycle containing 1-3 heteroatoms each independently selected from O, S and N, said heterocycle being optionally benzofused, and said phenyl or heterocycle being optionally further substituted by:
vi) halo;
vii) SOₚR⁴, where p is 0, 1 or 2;
viii) (C₁₋₅ alkyl)OH;
ix) (C₁₋₅ alkyl)CO₂H.

9. A compound of formula (I) as claimed in claims 1-8 where R³ is hydrogen, C(O)CH₃ or pyrimidine optionally substituted by chloro, bromo, SOₚCH₃, where p is 0, 1 or 2, (C₁₋₅ alkyl)OH and (C₁₋₅ alkyl)CO₂H.

10. A compound of formula (I) as claimed in claims 1-10 where R³ is 4-chloropyrimidinyl

11. A compound of formula (I) as claimed in claims 1-10 where R⁴ and R⁵ are hydrogen or C₁₋₆ alkyl.

12. A compound of formula (I) as claimed in claims 1-11 where R⁴ and R⁵ are hydrogen or C₁₋₃ alkyl.

13. A compound of formula (I) as claimed in claims 1-12 where R⁴ and R⁵ are CH₃.

14. A compound of formula (I) as claimed in claims 1-13 where R⁶ and R⁷ are hydrogen or CH₃.

15. A compound of formula (I) as claimed in claims 1-14 where R⁶ and R⁷ are hydrogen.

16. A compound of formula (I) as claimed in claims 1-15 or a pharmaceutically acceptable derivative thereof for use as medicament.

17. A compound of formula (I) as claimed in claims 1-15 or pharmaceutically acceptable derivative thereof as defined above, in the manufacture of a medicament for the treatment of a condition mediated by endothelin.

18. The use as claimed in claim 17 where the condition is mediated by endothelin ET_{A}

19. The use as claimed in claims 17-18 where the condition is selected from: restenosis, acute/chronic renal failure, pulmonary hypertension, systemic hypertension; benign prostatic hyperplasia, male erectile dysfunction, prostate cancer, metastatic bone cancer, congestive heart failure, stroke, subarachnoid haemorrhage, angina, atherosclerosis, cerebral and cardiac ischaemia, prevention of ischaemia/reperfusion injury (e.g. allografts), cyclosporin induced nephrotoxicity, glaucoma, radiocontrast nephropathy, diabetic neuropathy, allergy, restoration of organ perfusion in haemorrhagic shock, lipoprotein lipase related disorders, chronic obstructive pulmonary disease and hyaline membrane disease in newborn.

20. A method of treating conditions mediated by endothelin, which comprises administering to a patient in need of such treatment a therapeutically effective amount of a compound of formula (I) or a pharmaceutically acceptable derivative thereof.

21. A method of treating conditions mediated by endothelin ET_{A}, which comprises administering to a patient in need of such treatment a therapeutically effective amount of a compound of formula (I) or a pharmaceutically acceptable derivative thereof.

22. A method of treatment as claimed in claims 20-21 where the condition is selected from: restenosis, acute/chronic renal failure, pulmonary hypertension, systemic hypertension; benign prostatic hyperplasia, male erectile dysfunction, prostate cancer, metastatic bone cancer, congestive heart failure, stroke, subarachnoid haemorrhage, angina, atherosclerosis, cerebral and cardiac ischaemia, prevention of ischaemia/reperfusion injury (e.g. allografts), cyclosporin induced nephrotoxicity, glaucoma, radiocontrast nephropathy, diabetic neuropathy, allergy, restoration of organ perfusion in haemorrhagic shock, lipoprotein lipase related disorders, chronic obstructive pulmonary disease and hyaline membrane disease in newborn.

23. A pharmaceutical composition comprising a compound of formula (I), as claimed in claims 1-15, and a pharmaceutically-acceptable adjuvant, diluent or carrier.
